Europäisches Patentamt

European Patent Office

Office européen des brevets

(19)

(11) Numéro de publication: **0 1 1 2 7 3 6**
**A1**

(12) # DEMANDE DE BREVET EUROPEEN

(21) Numéro de dépôt: 83402198.2

(22) Date de dépôt: 15.11.83

(51) Int. Cl.³: **C 07 C 11/02,** C 07 C 7/177,
C 07 C 2/20, B 01 J 27/12

(30) Priorité: 10.12.82 FR 8220782

(43) Date de publication de la demande: 04.07.84
Bulletin 84/27

(84) Etats contractants désignés: BE DE GB IT NL

(71) Demandeur: **COMPAGNIE FRANCAISE DE RAFFINAGE
Société anonyme dite:, 5, rue Michel-Ange,
F-75781 Paris Cedex 16 (FR)**

(72) Inventeur: **Marty, Claude, 66, rue Guillemard, F-76600 Le
Havre (FR)**
Inventeur: **Engelhard, Philippe, 197, rue Félix Faure,
F-76600 Le Havre (FR)**

(74) Mandataire: **Jolly, Jean-Pierre et al, Cabinet BROT et
JOLLY 83, rue d'Amsterdam, F-75008 Paris (FR)**

(54) **Procédé d'oligomérisation de coupes d'oléfines légères en présence d'un catalyseur ou trifluorure de bore.**

(57) L'invention concerne l'oligomérisation de coupes d'oléfines.

Le procédé selon l'invention est caractérisé en ce que l'on
met du trifluorure de bore à l'état gazeux en contact avec ladite
coupe, en l'absence de tout support catalytique, dans des conditions et pendant une durée telles que l'isobutène et/ou le butadiène présents polymérisent, en ce que l'on sépare ensuite de
ladite coupe, de façon connue en soi, le trifluorure de bore et
les polymères d'isobutène et/ou de butadiène obtenus, et en ce
que l'on oligomérise les oléfines de la coupe ainsi purifiée en
présence d'un catalyseur au trifluorure de bore comprenant un
support catalytique.

Procédé d'oligomérisation de coupes d'oléfines légères
en présence d'un catalyseur au trifluorure de bore.

La présente invention concerne un procédé
d'oligomérisation de coupes d'oléfines légères ou de
mélanges de telles coupes.

On sait que le développement contemporain des
réactions de craquage des charges pétrolières rend
disponibles de grandes quantités d'oléfines possédant
de deux à huit atomes de carbone, telles que l'éthylène,
le propylène, les divers butènes, pentènes, hexènes,
heptènes et octènes. Parmi celles-ci, les coupes d'oléfines
à 3 et 4 atomes de carbone, dites coupes $C_3$, $C_4$ et/ou
coupes $C_3$ - $C_4$ sont particulièrement recherchées, car
plusieurs procédés de conversion permettent de les
valoriser. En particulier :

- leur oligomérisation permet d'obtenir, après
fractionnement, des produits de bases intéressants pour
la chimie, des solvants ou des huiles lubrifiantes,

- leur alkylation conduit à l'obtention de produits
possédant un indice d'octane élevé, qui entrent dans
la composition des supercarburants,

- leur dimérisation conduit également à l'obtention
de produits possédant un indice d'octane élevé, en ce
qui concerne le propylène, et à des oléfines ayant de
6 à 8 atomes de carbone, utiles comme plastifiants, en
ce qui concerne les coupes $C_4$ et/ou $C_3$ - $C_4$.

La grande disponibilité des oléfines de départ a
rendu nécessaire la mise au point de nombreux catalyseurs
de polymérisation. Parmi ceux-ci, on peut citer
principalement les catalyseurs à base d'acides protoniques
forts, tels que l'acide sulfurique, l'acide phosphorique,
l'acide fluorhydrique, les résines sulfoniques, les
acides de Lewis et, en particulier, le chlorure ou le
fluorure d'aluminium, le fluorure de bore, le fluorure
et le chlorure de zinc, le chlorure stannique, le
chlorure ferrique, en présence ou non d'un promoteur tel que l'eau, l'acide chlorhydrique, les alcools, les glycols ou l'éther anhydre.

Les recherches portant sur l'utilisation de catalyseurs phosphoriques ou de catalyseurs au trifluorure de bore ont montré que ces derniers sont les plus efficaces. Les catalyseurs au trifluorure de bore présentent en outre l'intérêt de permettre d'effectuer la réaction dans des conditions plus douces que les autres catalyseurs de polymérisation, tels que les catalyseurs à l'acide phosphorique ou fluorhydrique.

Ainsi, par exemple, le brevet français n° 1 346 135 décrit un procédé pour polymériser des hydrocarbures oléfiniques en présence d'un catalyseur comprenant une alumine à peu près anhydre, modifiée par le trifluorure de bore, qui permet d'obtenir un rendement en polymère relativement élevé. Dans ce brevet, aucune mention n'est faite, cependant, d'une quelconque possibilité de choix du support d'alumine en vue d'une orientation préférée vers tel ou tel effluent réactionnel, et aucun prétraitement de la charge n'est envisagé.

Ce type de procédé présente cependant un certain nombre d'inconvénients :

- d'une part, les coupes d'oléfines utilisées doivent être soumises à une purification préalable, car elles sont sensibles à la présence de nombreux polluants des catalyseurs, tels que le soufre, l'azote, l'isobutène ou le butadiène; mais, s'il est possible d'éliminer, au moins partiellement, les dérivés soufrés (mercaptans légers, $H_2S$, COS) et azotés (amines légères), il est beaucoup plus difficile d'éliminer l'isobutène et le butadiène, qui produisent des gommes et sont par là responsables de la désactivation rapide des catalyseurs;

- d'autre part, la conversion de l'isobutène conduit à la formation de composés ramifiés, qui sont généralement hors spécification (heptènes et octènes); divers prétraitements de charges d'oléfines légères ont certes été proposés dans la littérature (par exemple dans le brevet français n° 2 421 157), mais ils sont en général très coûteux et peu efficaces, car ils n'éliminent pas complètement l'isobutène et surtout ne permettent pas d'éliminer les traces de butadiènes;

- enfin, les procédés d'oligomérisation des oléfines utilisant comme catalyseur du trifluorure de bore sur support d'alumine produisent en trop grande quantité des produits lourds, ce qui nuit à l'économie du procédé.

La présente invention vise à remédier à ces inconvénients, en proposant un procédé d'oligomérisation des oléfines qui, d'une part, permet d'extraire sélectivement, sous forme de polymères, l'isobutène et le butadiène présents dans les coupes, par action du trifluorure de bore et en l'absence de tout support catalytique et, d'autre part, grâce au choix judicieux d'un support catalytique, permet d'orienter préférentiellement l'oligomérisation de coupes d'oléfines légères, notamment de propène, de butènes ou de leurs mélanges, vers des coupes à bas points d'ébullition, en limitant la formation de résidus de polymérisation à hauts points d'ébullition.

La Demanderesse a en effet établi que, de façon inattendue, dans un procédé d'oligomérisation d'oléfines en présence d'un catalyseur de polymérisation au trifluorure de bore et en l'absence de support catalytique tel que l'alumine, il est possible, dans une phase préliminaire, au cours de laquelle on utilise comme catalyseur du trifluorure de bore à l'état gazeux, de polymériser préférentiellement le butadiène et/ou l'isobutène présents, sans affecter profondément les autres oléfines des coupes à traiter, les polymères ainsi obtenus pouvant ensuite être facilement séparés par des moyens connus dans la

0112736

technique. Les polymères d'isobutène et de butadiène ainsi séparés des coupes à traiter sont alors aisément valorisés. En effet, après fractionnement et éventuellement hydrogénation, on obtient en fonction de leur température d'ébullition :

- des dimères ayant un indice d'octane élevé, utilisables dans les essences légères,

- des trimères intéressants pour la formulation d'essences lourdes, de solvants ou pour la pétrochimie,

- des tétramères qui, après hydrogénation, sont des donneurs de bases pour fluides hydrauliques,

- et enfin des polymères plus lourds, qui, également après hydrogénation, sont utilisables comme huile isoparaffinique.

Par ailleurs, la Demanderesse a établi que les catalyseurs au trifluorure de bore supportés par des alumines à faible surface spécifique et à faible volume poreux optimisent la formation d'oligomères à bas point d'ébullition et qu'une orientation préférentielle de la réaction vers l'obtention des polymères à plus ou moins haut point d'ébullition peut ainsi être obtenue par un traitement approprié du support catalytique.

L'invention a par conséquent pour objet un procédé d'oligomérisation de coupes d'oléfines légères ou de leurs mélanges, caractérisé en ce que l'on met du trifluorure de bore à l'état gazeux en contact avec ladite coupe d'oléfines, en l'absence de tout support catalytique et dans des conditions telles que l'isobutène et le butadiène présents polymérisent sélectivement, en ce que l'on sépare ensuite de ladite coupe, de façon connue en soi, le trifluorure de bore et les polymères d'isobutène et de butadiène obtenus, et en ce que l'on oligomérise les oléfines de la coupe ainsi purifiée en présence d'un catalyseur au trifluorure de bore comprenant un support catalytique, et notamment un support à base d'alumine. Pour obtenir des oligomères à bas point d'ébullition, on choisit en particulier un support à base d'alumine ayant une surface spécifique comprise entre 20 et 120 $m^2/g$ et un volume poreux compris entre 0,10 et 0,80 $cm^3/g$.

Un premier avantage important de ce procédé est que l'on utilise du trifluorure de bore aussi bien dans la phase préalable, pour polymériser le butadiène et l'isobutène, que dans la phase d'oligomérisation des oléfines, de sorte qu'il est possible d'alimenter en commun en trifluorure de bore les phases de prétraitement et d'oligomérisation et d'y recycler à volonté le trifluorure de bore séparé et récupéré à l'issue de chacune de ces phases.

Un second avantage de ce procédé réside dans le fait que le paramètre qui différencie les deux étapes de la réaction n'est pas lié à la température, au débit ou à la pression, mais à la présence ou non d'un support catalytique pour le trifluorure de bore. Le procédé selon la présente invention limite donc au strict minimum les difficiles problèmes de régulation thermique, qui sont propres à ce type de réaction, et supprime une source d'erreur de manipulation dans la conduite des unités industrielles utilisant le procédé selon la présente invention.

Un troisième avantage de ce procédé est de pouvoir adapter la production des oligomères aux unités industrielles qui suivent l'unité de production. En effet, le simple fait d'envoyer la charge prétraitée sur un support catalytique approprié (et sélectionné en fonction de sa surface spécifique et de son volume poreux) permet d'orienter la composition de la charge effluente vers des compositions contenant des oligomères à plus ou moins haut point d'ébullition suivant le besoin.

0112736

La phase de polymérisation du butadiène et de l'isobutène constituant le prétraitement peut être conduite dans les conditions suivantes :

- température :                                        O à 100°C,
- pression :                                           O,1 à 50 bars,
- concentration en trifluorure de bore de la charge à traiter : 500 à 10 000 ppm,
- vitesse spatiale horaire de la charge :             O,1 à 10 volumes par volume de masse de remplissage et par heure (v.v.h.).

On notera que ces conditions opératoires sont peu sévères et, en particulier, que la température peut être égale à la température ambiante ou proche de celle-ci.

Le contact entre la charge d'oléfines et le trifluorure de bore peut être effectué dans une enceinte, en présence d'une masse de remplissage inerte, destinée à accroître les surfaces de contact (morceaux de quartz, billes de verre, billes d'alumine, cailloux, etc...) et à éliminer les calories produites au cours de la réaction de polymérisation.

Il est ainsi possible d'éliminer sélectivement jusqu'à environ 95 % de l'isobutène et jusqu'à 100 % du butadiène présents dans les coupes traitées.

Après cette première phase, le trifluorure de bore peut être facilement séparé, par exemple à l'aide d'une distillation flash. Il est préférable de le séparer en premier lieu, afin d'éviter des réactions secondaires avec les oléfines.

Il est ensuite facile d'éliminer de façon connue, par exemple dans un débutaniseur, les polymères d'isobutène et de butadiène qui se sont formés, pour purifier ainsi les coupes $C_3$ ou $C_4$ ou leurs mélanges. Ces polymères ont d'ailleurs des possibilités importantes de valorisation sur le marché.

La phase d'oligomérisation proprement dite est ensuite conduite en présente de trifluorure de bore sur un support à base d'alumine, dont la surface spécifique est comprise entre 20 et 120 m$^2$/g et, de préférence, entre 40 et 100 m$^2$/g, et dont le volume poreux est compris entre 0,10 et 0,80 cm$^3$/g et, de préférence, entre 0,20 et 0,50 cm$^3$/g.

Les conditions d'oligomérisation pourront être, par exemple, les suivantes :

- température :                    0 à 130°C,
- pression :                       0,1 à 50 bars,
- vitesse spatiale horaire
  de la charge :                   0,5 à 10 volumes par
                                   volume de catalyseur
                                   et par heure (v.v.h.).

Pour préparer les supports catalytiques de la réaction d'oligomérisation, on pourra procéder à une calcination au four des alumines de départ pendant des durées variables, suivant les cas, jusqu'à ce que les caractéristiques désirées de surface spécifique et/ou de volume poreux soient atteintes.

Il est en effet connu (Encyclopedia of Chemical Technology, 3ème Édition, Volume 2, pages 222 à 228) que la température et la durée de calcination d'une alumine induisent des changements de structure de celle-ci, avec pour conséquence des modifications de sa surface spécifique et donc de son volume poreux. C'est ainsi qu'en traitant pendant une durée appropriée un support d'alumine à des températures qui varient entre 400 et 700°C, la surface spécifique résultante du support est comprise entre 350 et 150 m$^2$/g, ce qui favorise un contact optimum avec la charge et, par conséquent, accroît l'efficacité du catalyseur. En revanche, une calcination trop importante du support d'alumine diminue l'efficacité, puisque, avec l'élévation de la température, on obtient une diminution de la surface et du volume poreux.

A l'encontre de ces enseignements de la technique antérieure, la Demanderesse a établi que, sans abaisser notablement le rendement global de la réaction d'oligomérisation, en présence de trifluorure de bore supporté par de l'alumine, une sélectivité bien meilleure en oligomères à bas points d'ébullition peut être obtenue en limitant la surface spécifique et/ou le volume poreux du support catalytique, respectivement, entre 20 et 120 m$^2$/g et 0,10 et 0,80 cm$^3$/g, et que des valeurs appropriées de cette surface spécifique et du volume poreux peuvent être obtenues par calcination d'une alumine de départ telle qu'une alumine ou un hydrate d'alumine. On pourra utiliser n'importe quel hydrate d'alumine, qu'il soit préparé par précipitation d'un sel d'aluminium ou obtenu directement sous une forme naturelle ou commerciale, pourvu que ses caractéristiques de surface et de porosité soient supérieures ou égales à celles du support recherché et qu'il contienne principalement de l'alumine.

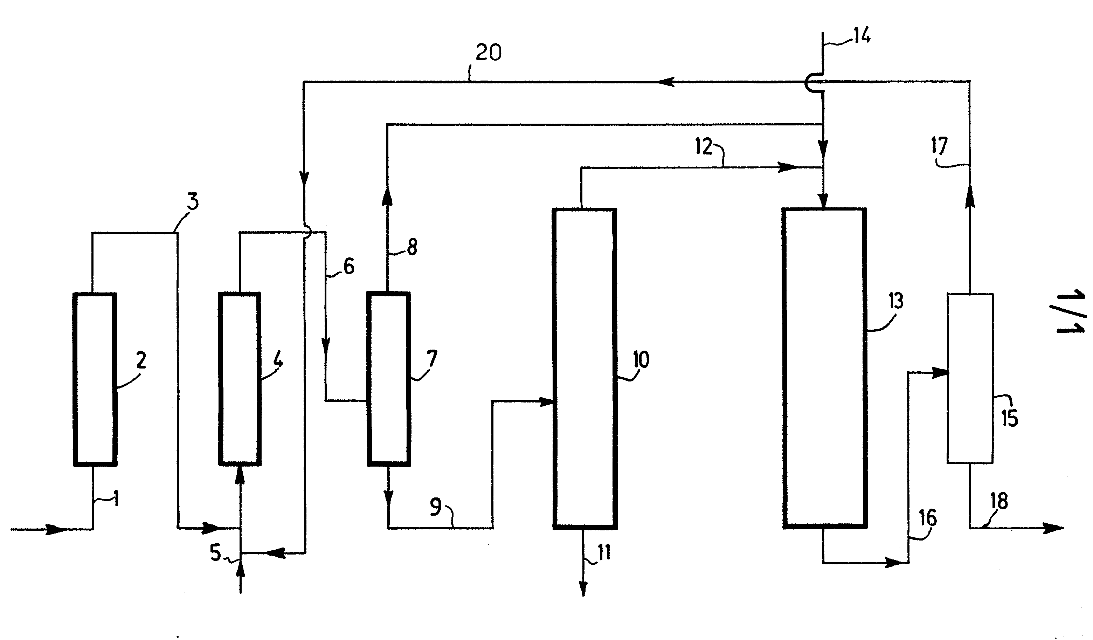

La figure unique du dessin annexé, qui n'a pas de caractère limitatif, est un schéma du procédé de traitement de coupes d'oléfines légères conforme à l'invention.

La charge fraîche d'oléfines (coupes $C_3$, $C_4$ et/ou $C_3$ - $C_4$) à traiter est introduite par la ligne 1 dans une enceinte 2, où elle est séchée sur des tamis moléculaires, en vue d'éliminer les traces d'humidité susceptibles de réagir avec le trifluorure de bore pour former des borates.

La charge ainsi séchée est conduite par la ligne 3 dans un réacteur 4, après avoir été mélangée avec du trifluorure de bore gazeux frais, qui emprunte la ligne 5. Compte tenu de la chaleur dégagée par la réaction de l'isobutène ou du butadiène avec le trifluorure de bore, celui-ci pourra avantageusement être dilué dans un gaz inerte tel que l'azote ou l'argon, ou encore dans

0112736

du butane ou du propane.

En vue d'accroître les surfaces de contact et de retenir la chaleur dégagée par la réaction, le réacteur 4 peut contenir une masse de contact inerte, constituée, par exemple, de morceaux de quartz, de billes de verre, de sable, de billes d'alumine, ou autres. Dans les conditions opératoires mentionnées ci-dessus, cette masse de contact peut être maintenue, par exemple, à une température d'environ 20°C.

Les effluents du réacteur 4 sont conduits par la ligne 6 dans un ballon de flash 7, dans lequel le trifluorure de bore gazeux est séparé en tête, par la ligne 8, de la coupe traitée, pour être dirigé vers le réacteur 13. Au fond du ballon de flash 7, la coupe d'oléfines traitée, contenant des polymères de butadiène et d'isobutène, est récupérée par la ligne 9 et acheminée vers une colonne de séparation 10.

On soutire en fond de colonne, par la ligne 11, l'isobutène et le butadiène polymérisés et, en tête de colonne, par la ligne 12, la coupe $C_4$ ou $C_3 - C_4$ ainsi purifiée, c'est-à-dire débarrassée de l'isobutène et du butadiène qui la polluaient originellement. La coupe ainsi traitée est ensuite acheminée par la ligne 12 dans un réacteur d'oligomérisation 13 contenant un support d'alumine préalablement saturé par un courant de trifluorure de bore, éventuellement dilué dans un gaz inerte tel que l'argon ou l'azote.

Pour maintenir l'activité du catalyseur dans le réacteur 13, un léger courant de trifluorure de bore est injecté dans celui-ci par la ligne 14 en même temps que la charge d'oléfines. Ce dernier est constitué par le trifluorure de bore séparé dans le ballon 7 et acheminé par la ligne ; un appoint de trifluorure de bore frais peut également être réalisé par la ligne 14.

Les effluents du réacteur 13 sont conduits à un séparateur 15 par la ligne 16. Dans le séparateur 15,

0112736

le trifluorure de bore présent dans les effluents est séparé des oligomères et recyclé par la ligne 17 vers la ligne 5. Les oligomères récupérés en fond de séparateur 15 par la ligne 18 sont ensuite fractionnés de façon usuelle en diverses coupes, en fonction de leurs températures d'ébullition.

Préalablement à ce fractionnement, si nécessaire, les oligomères provenant du séparateur 15 peuvent être décontaminés, afin d'éliminer les traces éventuelles dé trifluorure de bore. Le traitement de décontamination peut être réalisé à l'aide de moyens classiques, par exemple par passage sur alumine ou bauxite ou par lavage à la soude.

Les exemples suivants illustrent la mise en oeuvre de l'invention. Ils n'ont bien entendu aucun caractère limitatif.

### EXEMPLE 1

On traite, conformément à l'invention, une charge oléfinique venant de craquage catalytique et dont la composition est donnée dans le Tableau I ci-après.

0112736

## TABLEAU I

| Constituants | % en volume | % en poids |
|---|---|---|
| Hydrocarbures saturés | 53,4 | 55,6 |
| Oléfines dont : | 46,6 | 44,4 |
| Butène-1 | 6,1 | 6,3 |
| Isobutène | 8,5 | 8,9 |
| Butène-2-trans | 9,7 | 10,1 |
| Butène-2-cis | 5,8 | 6,0 |
| Propène | 16,5 | 13,1 |
| Butadiène 1-3 | 500 ppm | 600 |

Les conditions de traitement dans le réacteur 4 sont les suivantes :

- température :  20°C,
- pression :  30 bars,
- vitesse spatiale horaire de la charge :  3 v.v.h.,
- concentration de trifluorure de bore (rapporté à la charge) : 2 000 ppm,
- nature de la masse de contact : 50 cm$^3$ de billes de verre de 2 mm de diamètre.

L'effluent gazeux sortant de la colonne de séparation 10, par la ligne 12, a la composition donnée au Tableau II ci-après :

0112736

TABLEAU II

| Constituants | % en volume | % en poids |
|---|---|---|
| Hydrocarbures saturés | 61 | 62 |
| Oléfines dont : | 39 | 37,4 |
| Butène-1 | 6,7 | 6,9 |
| Isobutène | 0,8 | 0,9 |
| Butène-2-trans | 9,3 | 9,7 |
| Butène-2-cis | 5,7 | 5,9 |
| Propène | 16,5 | 14,0 |
| Butadiène | 200 ppm | 200 ppm |

Le prétraitement a permis un taux d'élimination spécifique de chaque oléfine présenté dans le Tableau III ci-dessous :

TABLEAU III

| Oléfines | Taux d'élimination spécifique (% en poids) |
|---|---|
| Butène-1 | 3 |
| Isobutène | 92 |
| Butène-2 | 16 |
| Propène | 5 |
| Butadiène | 70 |

Ce traitement a donc eu pour effet d'éliminer 92 % de l'isobutène de la charge et 70 % du butadiène.

La charge épurée, dont la composition est donnée au Tableau II, est ensuite traitée dans le réacteur d'oligomérisation 13. Ce réacteur contient un catalyseur au trifluorure de bore sur support d'alumine à faible

surface spécifique et à faible volume poreux. Ce catalyseur a été préparé de la façon suivante :

On est parti d'une alumine commerciale, dont la structure était principalement sous forme $\eta$ et dont les caractéristiques étaient les suivantes :

- surface spécifique (Sp) :     400 $m^2$/g,
- volume poreux (Vp) :     0,46 $cm^3$/g,
- rayon des pores (Rp) :     20 $\overset{\circ}{A}$,
- granulométrie (après tamisage) :     2 - 2,5 mm,
- densité apparente :     0,80 g/$cm^3$.

Cette alumine a été calcinée au four pendant 24 heures, à une température de 1 000°C. Après calcination, cette alumine présentait une structure $\theta + \alpha$. Sa surface spécifique était égale à 43 $m^2$/g et son volume poreux à 0,20 $cm^3$/g.

Le support de catalyseur ainsi préparé a été placé dans le réacteur 13 et on l'a saturé par passage d'un courant gazeux de trifluorure de bore dilué à 10 % dans l'azote, dans les conditions suivantes :

- durée :     24 heures,
- température :     130°C,
- pression :     25 bars,
- débit :     2,5 nl/h pour 50 $cm^3$ de support dilué dans 50 $cm^3$ de billes de verre de même granulométrie.

Cette opération terminée, on a procédé à l'oligomérisation de la charge prétraitée dans le réacteur 13 dans les conditions suivantes :

- pression constante :     30 bars,
- concentration en $BF_3$ constante :     2 000 ppm (par rapport à la charge),
- température :     75°C,
- vitesse spatiale horaire de la charge :     6 v.v.h.

- 14 -

0112736

A la sortie du réacteur 13, les effluents sont recueillis dans le séparateur 15, où l'on obtient, d'une part, une phase gazeuse contenant les produits non transformés et le trifluorure de bore évacués par la ligne 17 et recyclés vers la ligne 5 et, d'autre part, une phase liquide contenant les produits d'oligomérisation. Ces derniers sont ensuite fractionnés pour être séparés en quatre coupes :

- coupe 60 - 120°C,
- coupe 120 - 220°C,
- coupe 220 - 250°C,
- coupe 250 - 320°C,

et un résidu de point d'ébullition supérieur à 320°C.

Le cas où la charge n'est pas soumise à un prétraitement au trifluorure de bore gazeux a été également étudié et le tableau IV ci-après indique les rendements et sélectivités en oligomères obtenus avec et sans prétraitement :

TABLEAU IV

| Rendement en oligomères formés (en g/100 g d'oléfines) | Sans prétraitement | Avec prétraitement |
|---|---|---|
| | 80 | 83 |
| Sélectivité des oligomères (% en poids) | | |
| Coupe 60 - 120°C | 20 | 40 |
| Coupe 120 - 220°C | 46,6 | 40,8 |
| Coupe 220 - 250°C | 12,7 | 6,9 |
| Coupe 250 - 320°C | 13,2 | 9,3 |
| Résidu + 320°C | 7,5 | 3 |

On observe, dans le présent cas d'oligomérisation, un taux d'élimination spécifique de chaque oléfine présenté dans le Tableau V ci-après :

BAD ORIGINAL

0112736

TABLEAU V

| Oléfines | Taux d'élimination de chaque oléfine (% en poids) | |
|---|---|---|
| | Sans prétraitement | Avec prétraitement |
| Butène-1 | 93 | 92 |
| Isobutène | 100 | 100 |
| Butènes-2 | 71 | 68 |
| Propène | 94 | 95 |
| Butadiène | 100 | 100 |

EXEMPLE 2

On traite conformément à l'invention une charge ayant la composition suivante (Tableau VI) :

TABLEAU VI

| Constituants | % en volume | % en poids |
|---|---|---|
| Hydrocarbures saturés | 54,1 | 56,3 |
| Oléfines dont : | 45,9 | 43,7 |
| Propène | 16,4 | 13,0 |
| Butène-1 | 6,1 | 6,2 |
| Isobutène | 8,0 | 8,4 |
| Butène-2-trans | 9,6 | 10,1 |
| Butène-2-cis | 6,8 | 6,0 |
| Butadiène | 500 ppm | 600 ppm |

Cette charge est traitée dans le réacteur 4 dans des conditions identiques à celles de l'Exemple 1 :

0112736

- température :                         20°C,

- pression :                           30 bars,

- vitesse spatiale horaire
  de la charge :                       3 v.v.h.,

- concentration constante en
  trifluorure de bore
  (rapportée à la charge) :            2 000 ppm

- nature de la masse de
  contact :                            50 ml de billes de
                                       verre de 2 mm de
                                       diamètre.

Le Tableau VII suivant donne la composition de la charge prétraitée.

### TABLEAU VII

| Constituants | Composition | |
|---|---|---|
| | % en volume | % en poids |
| Hydrocarbures saturés | 62,1 | 63,8 |
| Oléfines dont : | 37,9 | 36,2 |
| Propène | 16,5 | 14,0 |
| Butène-1 | 6,6 | 6,7 |
| Isobutène | 0,4 | 0,4 |
| Butène-2-trans | 8,9 | 9,4 |
| Butène-2-cis | 5,5 | 5,7 |
| Butadiène | 0 | 0 |

On constate que tout le butadiène de la charge de départ a été éliminé. 92 % de l'isobutène a également été éliminé de la charge.

La charge épurée, dont la composition est donnée au Tableau VII, est ensuite traitée dans le réacteur 13 dans les mêmes conditions que dans l'Exemple 1, mais avec un catalyseur ayant un support d'alumine

0112736

de structure $\eta$ , de surface spécifique égale à 201 m$^2$/g et de volume poreux égal à 0,41 cm$^3$/g. Cette alumine a été préparée par calcination pendant 4 heures, à 600°C, de l'alumine commerciale dont les caractéristiques ont été données à l'Exemple 1.

Après traitement dans le réacteur 13 et séparation des effluents dans le séparateur 15, on recueille une phase liquide qui, comme à l'Exemple 1, est fractionnée en différentes coupes.

On traite également dans le réacteur 13 une charge identique qui n'a pas été prétraitée dans le réacteur 4.

On effectue enfin un prétraitement et un traitement identiques en remplaçant l'alumine par celle qui a été calcinée dans les conditions de l'Exemple 1.

Les rendements en oligomères et les sélectivités obtenues dans ces trois cas sont indiqués au Tableau VIII ci-après.

## TABLEAU VIII

| | Alumine à forte surface (alumine $\eta$) | | Alumine à faible surface (alumine $\Theta + \alpha$) |
|---|---|---|---|
| | Sans prétraitement | Avec prétraitement | Avec prétraitement |
| Rendement en oligomères (en g/100 g d'oléfines) | 92 | 93 | 85 |
| Sélectivité des oligomères (% en poids) | | | |
| Coupe 60 – 120°C | 14,9 | 20 | 38 |
| Coupe 120 – 220°C | 36,7 | 40 | 41,2 |
| Coupe 220 – 250°C | 15,4 | 14,4 | 7,8 |
| Coupe 250 – 320°C | 19,8 | 17,6 | 9,2 |
| Résidu + 320°C | 13,2 | 8,0 | 3,8 |

- 18 -

0112736

- 19 -

Le taux de transformation de chaque oléfine relatif aux trois cas d'oligomérisation ci-dessus est présenté dans le Tableau IX ci-après :

## TABLEAU IX

| Oléfines | Taux de transformation de chaque oléfine (% en poids) | | |
|---|---|---|---|
| | Alumine à forte surface | | Alumine à faible surface |
| | Sans prétraitement | Avec prétraitement | Avec prétraitement |
| Butène-1 | 100 | 98 | 93 |
| Isobutène | 100 | 100 | 100 |
| Butènes-2 | 74 | 88 | 68 |
| Propène | 100 | 100 | 95 |
| Butadiène | 100 | 100 | 100 |

- 20 -

0112736

0112736

Ces résultats montrent une augmentation sensible de la sélectivité en coupe légère 60 - 130°C, avec une très nette diminution de la sélectivité en résidu ()320°C), lorsque, conformément à l'invention, on opère avec un catalyseur de faible surface spécifique, après un prétraitement de la charge à l'aide de trifluorure gazeux, en vue de polymériser sélectivement le butadiène et l'isobutène qu'elle contient.

## REVENDICATIONS

1.- Procédé d'oligomérisation de coupes d'oléfines légères ou de leurs mélanges, caractérisé en ce que l'on met du trifluorure de bore à l'état gazeux en contact avec ladite coupe, en l'absence de tout support catalytique, dans des conditions et pendant une durée telles que l'isobutène et/ou le butadiène présents polymérisent, en ce que l'on sépare ensuite de ladite coupe, de façon connue en soi, le trifluorure de bore et les polymères d'isobutène et/ou de butadiène obtenus, et en ce que l'on oligomérise les oléfines de la coupe ainsi purifiée en présence d'un catalyseur au trifluorure de bore comprenant un support catalytique.

2.- Procédé selon la revendication 1, caractérisé en ce que la coupe d'oléfines à traiter et le trifluorure de bore gazeux sont mis en contact en présence d'une masse de remplissage inerte.

3.- Procédé selon l'une des revendications 1 et 2, caractérisé en ce que la coupe d'oléfines à traiter et le trifluorure de bore gazeux sont mis en contact à une température comprise entre 0 et 100°C et sous une pression comprise entre 0,1 et 50 bars.

4.- Procédé selon l'une des revendications 1 à 3, caractérisé en ce que, après mise en contact de la coupe d'oléfines à traiter et du trifluorure de bore à l'état gazeux, ce dernier est éliminé de ladite coupe préalablement à la séparation des polymères d'isobutène et/ou de butadiène obtenus.

5.- Procédé selon la revendication 4, caractérisé en ce que la séparation du trifluorure de bore de la charge traitée comprend une phase de distillation flash.

6.- Procédé selon l'une des revendications 1 à 5, caractérisé en ce que l'oligomérisation de la coupe ainsi purifiée est réalisée en présence d'un catalyseur au trifluorure de bore comprenant un support catalytique actif à base d'alumine.

7.- Procédé selon la revendication 6, caractérisé en ce que, pour orienter la sélectivité de l'oligomérisation vers la production d'effluents di- ou trimérisés, la phase d'oligomérisation des oléfines s'effectue en présence d'un catalyseur au trifluorure de bore comprenant un support à base d'alumine dont la surface spécifique est comprise entre 20 et 120 $m^2$/g et, de préférence, entre 40 et 100 $m^2$/g, et dont le volume poreux est compris entre 0,10 et 0,80 $cm^3$/g et, de préférence entre 0,20 et 0,50 $cm^3$/g.

8.- Procédé selon l'une des revendications 1 à 7, caractérisé en ce que la phase d'oligomérisation s'effectue en présence de trifluorure de bore sur un support à base d'alumine dont la surface spécifique et le volume poreux ont été ajustés par calcination d'un support brut.

9.- Procédé selon l'une des revendications 1 à 8, caractérisé en ce que la phase d'oligomérisation des oléfines est conduite à une pression comprise entre 0,1 et 50 bars, et à une température comprise entre 0 et 130°C.

10.- Procédé selon l'une des revendications 1 à 9, caractérisé en ce que le trifluorure de bore séparé à l'issue de la phase de polymérisation du butadiène et de l'isobutène et/ou à l'issue de la phase d'oligomérisation des oléfines est recyclé à l'une et/ou l'autre de ces phases.

11.- Procédé selon l'une des revendications 1 à 10, caractérisé en ce que la mise en contact de la coupe d'oléfines avec du trifluorure de bore gazeux et l'oligomérisation des oléfines de la coupe en présence de trifluorure de bore sur support catalytique s'effectuent dans des conditions opératoires sensiblement identiques.

1/1

011273G

0112736

Office européen
des brevets

**RAPPORT DE RECHERCHE EUROPEENNE**

Numéro de la demande

EP 83 40 2198

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| X | FR-A-2 504 121 (COMPAGNIE FRANCAISE DE RAFFINAGE) * Revendications * ----- | 1,6-9 | C 07 C 11/02 C 07 C 7/177 C 07 C 2/20 B 01 J 27/12 |

**DOMAINES TECHNIQUES RECHERCHES (Int. Cl. 3)**

C 07 C 11/00
C 07 C 7/00
C 07 · C 2/00

Le présent rapport de recherche a été établi pour toutes les revendications

| Lieu de la recherche | Date d'achèvement de la recherche | Examinateur |
|---|---|---|
| LA HAYE | 22-02-1984 | VAN GEYT J.J.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant